Europäisches Patentamt

European Patent Office

Office européen des brevets

(11) Publication number: **0 148 551**
**A2**

(12) **EUROPEAN PATENT APPLICATION**

(21) Application number: **84303614.6**

(22) Date of filing: **29.05.84**

(51) Int. Cl.⁴: **G 01 N 21/64, G 01 N 33/48**

(30) Priority: **27.05.83 JP 92489/83**

(43) Date of publication of application: **17.07.85**
**Bulletin 85/29**

(84) Designated Contracting States: **CH DE FR GB IT LI NL**

(71) Applicant: **FUJIREBIO KABUSHIKI KAISHA also trading as FUJIREBIO INC., 6-7, Shimoochiai 4-chome Shinjuku-ku, Tokyo 161 (JP)**

(72) Inventor: **Nakae, Taiji, 570-3, Awakubo, Isehara-shi Kanagawa-ken (JP)**
Inventor: **Ryo, Eisaku, 122-1, Shirane, Isehara-shi Kanagawa-ken (JP)**

(74) Representative: **Silverman, Warren et al, HASELTINE LAKE & CO. Hazlitt House 28 Southampton Buildings Chancery Lane, London WC2A 1AT (GB)**

(54) **Method for measuring membrane fusion.**

(57) A simple and accurate method of measuring membrane fusions which has application in drug administration comprises fusing liposomes containing porins with cation-loaded liposomes lacking porins, mixing the reaction mixture obtained with an ionophore and with a fluorescent dye that is sensitive to the negative potential of the liposomes lacking porins induced by the ionophore and measuring the change in fluorescence intensity of the dye which has occured as a measure of the extent of membrane fusion.

EP 0 148 551 A2

ACTORUM AG

## METHOD OF MEASURING MEMBRANE FUSION

This invention relates to a method of measuring membrane fusions among liposomes made of phospholipids whether or not the latter are bound to protein.

Liposomes made of phospholipids, e.g. phosphatidylcholine, have been used by a number of investigators as a good model for studying the structure and the function of biological membranes. Recently, a number of workers have proposed that liposomes be used as vehicles for drug delivery. If the liposomes can be fused with cell membranes in vivo, the scope for administration of drugs in the form of liposome drug combinations will be unlimited. For example, if liposomes containing anti-tumor drug(s) in their intraliposomal space can be delivered specifically to tumor cells and be fused with them, the adverse effects caused by the drug can be minimized. Therefore there has developed a need for establishing working methods that can be used in research into the mechanism of membrane fusion. However, there is little reported work concerning quantitative measurement into membrane fusion, and therefore knowledge of the mechanism of membrane fusion is poor. In one method, the fluorescence intensity of the internal contents of two fused liposomes upon mixing has been monitored. More particularly fusion of liposomes containing terbium ions ($Tb^{3+}$) and liposomes containing dipicolinic acid (DPA) resulted in an enhancement of fluorescence emission through the production of $Tb(DPA)_3^{3-}$. Because the intravesicular contents, Tb and DPA leak out from liposomes and external EDTA influxes into liposomes during membrane fusion, this method cannot be used to measure membrane fusion quantitatively.

It is an object of the invention to provide an accurate and simple method of measuring membrane fusion.

According to the present invention, there is provided a method of measuring membrane fusion which

0148551

comprises fusing liposomes containing porins with cation-loaded liposomes lacking porins, mixing the reaction mixture obtained with an ionophore and with a fluorescent dye that is sensitive to the negative potential of liposomes lacking porins induced by the ionophore and measuring the change in fluorescence intensity of the dye which has occurred as a measure of the extent of membrane fusion.

More specifically the present invention provides for measurement of membrane fusion by fusing liposomes containing porins (hereinafter termed porin-liposomes) with cation loaded, preferably $K^+$-loaded, liposomes lacking porin (hereinafter termed porinless-liposomes). The existence of unfused porinless-liposomes at any time is quantified by determination of the change in extent of quenching of fluorescence of the fluorescent dye upon dilution of the mixture with cation-containing medium in the presence of an ionophore, the cation being different from that used to load the porinless liposome, e.g. $Na^+$ to $K^+$ of the latter. The porin-containing liposomes (porin-liposomes) do not accumulate the fluorescent dye due to the instantaneous equilibrium of its two types of cation across the membranes via porin pores.

Many phospholipids may be used to produce the two types of liposome. They include phosphatidylcholine, phosphatidylserine, phosphatidylinositol, phosphatidylethanolamine, phosphatidic acid, cardiolipin and sphingomyelin. Two or more phospholipids can be mixed to prepare each liposome type. It is not important whether or not the phospholipids species for the two types of liposomes are the same or not.

Other materials than the phospholipid that alter the physical properties of the membrane, for example cholesterol, can be added to the material forming the liposomal membranes.

The pore forming porin material is composed of three identical subunits. The porin is obtainable in pure form from the outer membrane of gram-negative bacteria such as <u>Escherichia</u> <u>coli</u> and <u>Salmonella</u>, and the outer membrane of mitochondria. Porins are transmembraneous proteins that form pores in the membrane.

Both types of liposomes to be used are usually spherical and their intraliposomal space is filled with aqueous solution. The internal solution of the porinless-liposome should contain the cation which is specific for the ionophore to be used. Such cations include hydrogen ion, alkali metal ions, i.e. potassium ion, sodium ion, rubidium ion, caesium ion and lithium ion and alkaline earth metal ion, in particular calcium ion.

A salt of one of these ions is entrapped in the internal space of the liposomes. Such salts may be inorganic salts, for example chloride, nitrate, sulphate, phosphate or carbonate or organic salts, for example acetate, citrate, succinate and gluconate. The salt concentration should be 20 to 200mM.

The two types of liposomes can be prepared by following conventional methods. For example, phospholipids dissolved in an organic solvent such as chloroform or ether are placed in a spitz tube and dried to form a film. The dried phospholipid film is suspended in the above salt solution and sonicated. For the preparation of porin-liposomes, phospholipid film dried as above is resuspended in distilled water by sonic oscillation and then porins are added. The mixture is dried again at say $45^{\circ}C$ under an inert gas stream, for example a nitrogen stream, and kept in an evacuated desiccator. Then the dried film is resuspended in the above salt solution by oscillation.

Membrane fusion between two types of liposome can be initiated by added a fusogen, for example calcium ion, polyethylene glycol or Sendai virus, or by imposing an electric field. Calcium-induced membrane fusions can be terminated by dilution of the mixture or by addition of chelates such as EDTA or EGTA.

After termination of membrane fusion, the reaction mixtures are diluted with a medium free from the cations of porin-lacking liposome and containing a different cation, a fluorescent dye and an ionophore.

Many kinds of ionophore can be used when carrying out method of the invention. These include valinomycin (ionophore for potassium ion, rubidium ion and caesium ion), gramicidin A (ionophore for hydrogen ion, lithium ion, sodium ion and potassium ion), nonactin, monactin and dicyclohexyl 18-crown-6 (ionophores for potassium ion and sodium ion), cryptate 211 (ionophore for calcium ion, lithium ion and sodium ion), nigericin (ionophore for calcium ion), monensin (ionophore for sodium ion), A23187 and X537A (ionophores for calcium ion) and alamethecin (ionophore for potassium ion). The optimum concentrations of these ionophores vary depending on the ionophore used, the concentrations of cations and the character of the two types of liposomes to be used. Therefore, the nature and quantities of a particular combination of ionophore and ions should be determined in advance.

The fluorescent dyes to use include cyanine dyes, for example 3,3'-dipropylthiodicarbocyanine iodide (diS-$C_3$-(5)), 3,3'-dipropyloxadicarbocyanine iodide (diO-$C_3$-(5)) and 3,3'-dipropylindotricarbocyanine iodide (diI-$C_5$-(7)), merocyanine dyes, for example 5-[3-sodium sulphopropyl-2(3H)-benzoxazolylidene)-2-butenylidene]-1,3-dibutyl-2-thiobarbituric acid (Merocyanine 540), 5-[(1-γ-triethylammonium sulphopropyl-4-(1H)-quinolylidene]-3-ethylrhodamine (WW375), 5-[(3-γ-sodium sulphopropyl-2(3H)-thiazolinylidene)-2-butenylidene]-1,3-dibutyl-2-thiobarbituric acid and 5-[(3,3-dimethyl-1-γ-sodium sulphopropyl-2(3H)-indolylidene)-2-butenylidene]-3-ethyl rhodamine, and oxonal dyes, for example bis-[1,3-dibutyl-barbituric acid-(5)]-pentamethineoxonol (diBA-$C_4$-(5)),

bis-[3-phenyl-rhodamine-(5)] methinoxonol and bis-[3-γ-sodium sulphopropyl-rhodamine-(5)] methinoxonol. A typlical suitable concentration for the fluorescent dyes is one of the order of $10^{-6}$M.

The ionophores and the fluorescent dyes will generally be dissolved in an organic solvent, for example ethanol or acetone.

The change in fluorescence intensity can be monitored using a fluorophotometer. The optimum excitation and emission wave lengths to be used depend on the fluorescent dyes to be employed.

On dilution of the reaction mixture with a cation-containing solution, say a solution containing $Na^+$, as well as the ionophore and the fluorescent dye, the fluorescence intensity of the dye initially decreases and then recovers exponentially. The plots of the logarithms of the differences of the fluorescence intensities from that at equilibrium versus time are straight line plots. The extent of the maximum fluorescence quenching representing the ionophore-induced potential of porinless-liposomes can be obtained by extrapolating the line to time 0. As the extent of the fluorescence quenching is a function of the concentration of porinless-liposomes, the decrease in the concentration of porinless-liposomes due to the fusion thereof with porin-liposomes results in a decrease of the quenching of the dye. Accordingly the extent of membrane fusions can be determined from the extent of fluorescence quenching of the dye.

The principle of the assay by the method of the invention may be explained with reference to the use of valinomycin and diS-$C_3$-(5) as the ionophore and the fluorescent dye, respectively.

Upon addition of valinomycin to a solution containing $K^+$-loaded porinless-liposomes, the potassium ions efflux from the liposomes, because the potassium ion concentration of the solvent medium is lower than that of the internal solution of the liposomes. Since the chemical potential of the liposome interior becomes negative, diS-C$_3$-(5), having the positive charge, enters the liposomes. The fluorescence of the dye which enters the liposome is quenched. Porin-liposomes do not accumulate diS-C$_3$-(5) due to the instantaneous equilibrium of potassium and sodium ions across the membranes via porin pores. As a result of the fusion of porin-liposomes with K$^+$-loaded porinless-liposomes, loaded potassium ion will have efluxed immediately through the porin pores. Accordingly, the dye does not accumulate in porin liposomes which have become fused and hence no fluorescence quenching attributable to such fused porin-liposomes can be measured.

The method of this invention enables membrane fusions induced by many kinds of fusogens to be measured simply and accurately. Therefore the method of this invention finds practical application in the elucidation of the mechanism of the membrane fusion, in the application of the liposomes to drug administration and in investigations into fusogens.

The following examples illustrate this invention:

### EXAMPLE 1

1.8 mg of phosphatidylserine and 0.45 mg of phosphatidylcholine both dissolved in chloroform, were mixed in a spitz tube and the solvent was removed under a nitrogen gas stream at ambient temperature. The tube was placed in an evacuated desiccator containing silica gel for one hour to dry the phospholipid mixture completely.

150 µl of distilled water were added to the tube and the phospholipid film attached to the tube was scraped off by the use of a glass rod. The suspension of lipid in water was subjected to sonic vibration for 2 min using the microtip of a Bransonic Sonifier 200. In this way porinless liposome was produced. To prepare porin-liposomes, 110 µg porin in distilled water were added to a suspension produced as thus described. The mixture produced was dried at 40°C under a nitrogen gas stream. The tube was placed in a desiccator as described above for about one hour. The dried film produced was resuspended in 150 μl of 10mM Tris-HCl buffer (pH 8.0) containing 100 mM potassium gluconate by application of sonic vibration for 10 min using the above procedure. The porinless-liposomes initially produced and the porin-liposomes subsequently produced by the aforesaid procedure sized about 250 to 500 Å in diameter.

10 μl of 100 mM potassium gluconate/10 mM Tris-HCl buffer (pH 8.0) containing calcium ion were added to a mixture of 10 μl each of porin- and porinless-liposomes and the reaction mixture was incubated at a fixed temperature.

The reaction was terminated by diluting the reaction mixture with 280 μl of 10mM potassium gluconate/10 mM Tris-HCl buffer (pH 8.0).

20 μl of the mixture thus produced were added to a mixture of 25 ml of 100 mM sodium gluconate/10 mM Tris-HCl buffer (pH 8.0) and 2 μl of diS-C$_3$-(5) dissolved in ethanol in a cuvette. After one minute, 8 μl of 12 μM valinomycin dissolved in acetone were added and mixed in quickly. The change in the fluorescence intensity which occurred was monitored at 670 nm (slit width was 6 nm) with an excitation wavelength at 620 nm (slit width was 6 nm) using a Hitachi 650-10 M fluorescence spectrophotometer.

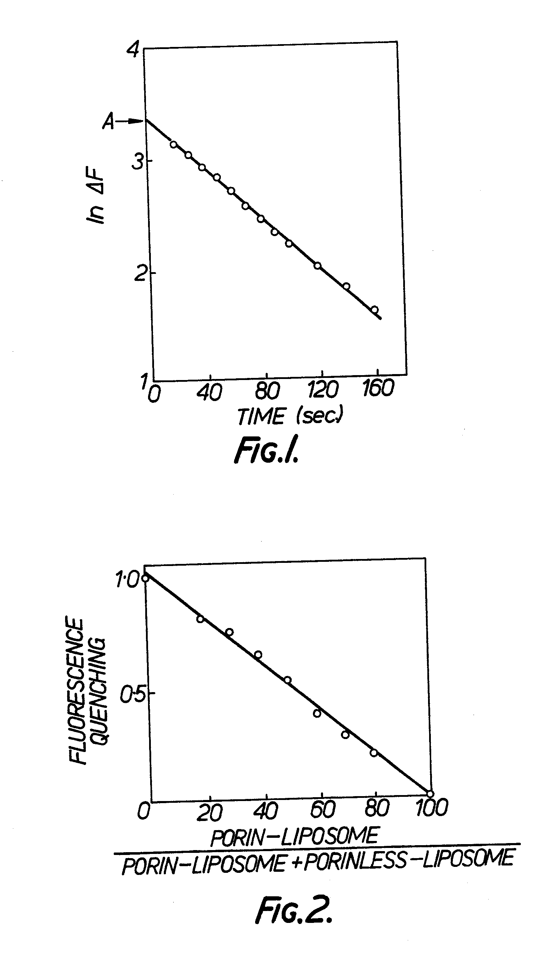

Figure 1 of the accompanying drawings shows the change with time of the fluorescence intensity after adding valinomycin. More particularly, the logarithms of the differences of fluorescence intensities between a particular time and at equilibrium (Pn △F) were plotted against time. The maximum fluorescence quenching indicated by the point A in Figure 1 is obtained by extrapolating the line to time 0.

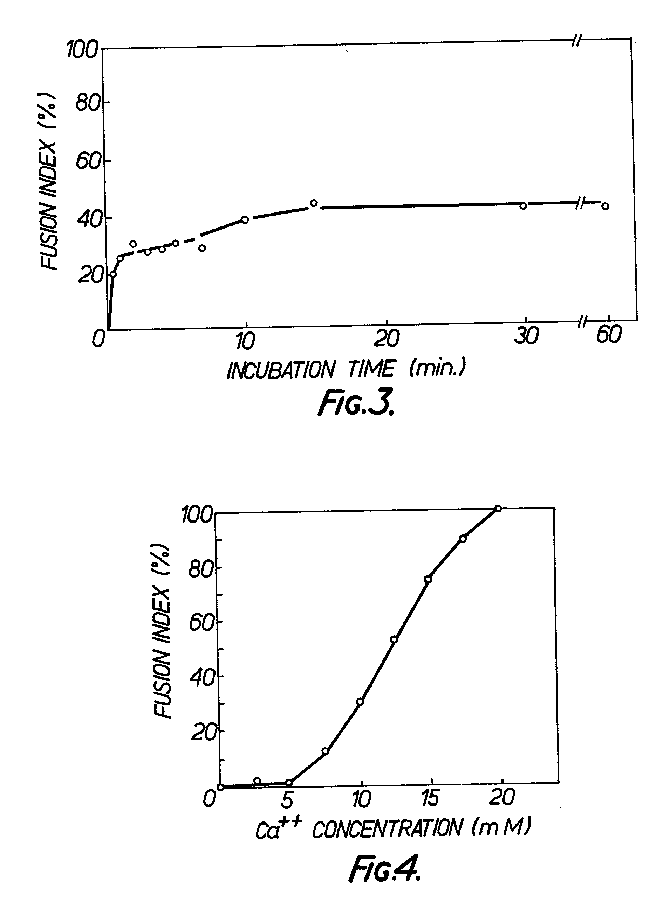

Figure 2 of the accompanying drawings shows the relationship between the quantity of porin-liposomes and the extent of fluorescence quenching. In repetitions of the above procedure, the porin-liposomes and the porinless-liposomes were mixed in various ratios and the fluorescence intensity was recorded as described above. The extent of fluorescence quenching by the porinless-liposomes only was normalized to 1.0. The extent of fluorescence quenching was shown to be linearly related to the population of porinless-liposomes. The membrane fusions induced by 10 mM $Ca^{++}$ were carried out at ambient temperature. Figure 3 of the accompanying drawings shows the time course of membrane fusions with the two aforesaid types of liposomes. The extent of membrane fusion can be expressed by the fusion index computed according to the following equation.

$$\text{Fusion Index (\%)} = (1 - \frac{\Delta F}{\Delta F_o}) \times 100$$

$F_o$: the extent of fluorescence quenching of the dye without a fusogen.

F : the extent of fluorescence quenching of the dye with a fusogen.

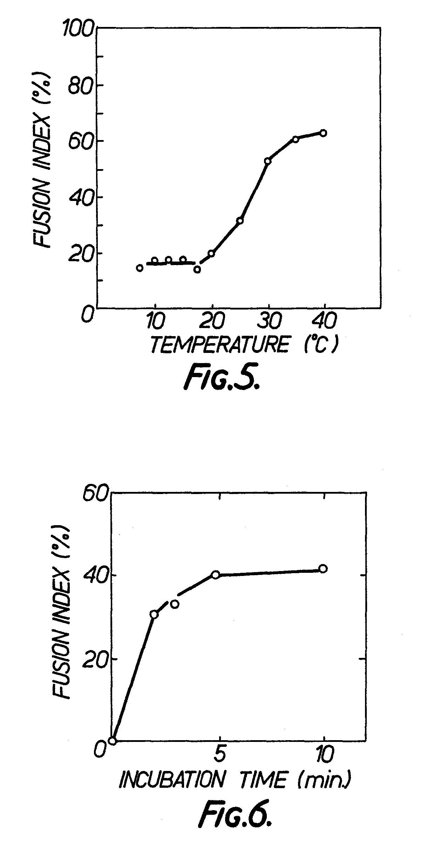

Figure 4 shows the effect of the presence of calcium ions on the membrane fusions. The reaction mixture was held at ambient temperature for 3 minutes before determining the fusion index.

Finally, Figure 5 of the accompanying drawings shows the effect of incubation temperature on membrane fusions. For each determination, the reaction mixture contained 12.5 mM calcium and was incubated for 3 min.

## EXAMPLE 2

In this example polyethylene glycol 4000 (PEG-4000) was used as a fusogen instead of calcium ions.

A mixture containing 10 μl each of porin- and porinless-phosphatidylcholine liposomes produced in analogous manner to that described in Example 1 was mixed with 40 μl of 37.5% w/v of PEG-4000 in 100 mM potassium gluconate/10 mM Tris-HCl buffer (pH 8.0). The mixture was incubated at 35°C and diluted with 540 μl of the same buffer. Fluorescence intensity was measured as described in Example 1 using 20 μl of the mixture. Figure 6 of the accompanying drawings shows how the extent of PEG-induce membrane fusions varied with time.

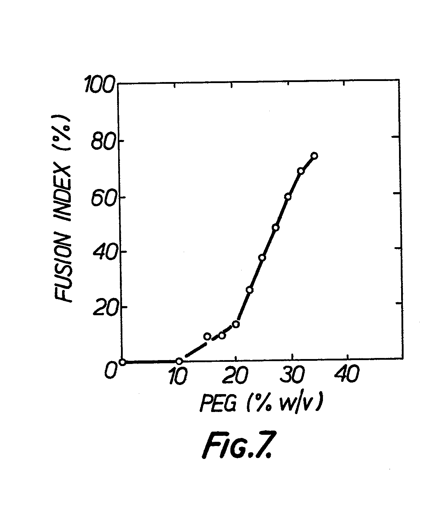

Figure 7 of the accompanying drawings shows the relationship between PEG concentration and the extent of membrane fusions. Reaction was carried out at 35°C for 10 min before fluorescence quenching (and hence fusion index) was determined.

CLAIMS:

1.     A method of measuring membrane fusion which comprises fusing liposomes containg porins with cation-loaded liposomes lacking porins, mixing the reaction mixture obtained with an ionophore and with a fluorescent dye that is sensitive to the negative potential of the liposomes lacking porins induced by the ionophore and measuring the change in fluorescence intensity of the dye which has occurred as a measure of the extent of membrane fusion.

2.     A method as claimed in claim 1, wherein the liposomes containing porin and the liposomes lacking porin are the same or different and formed of one or more phopholipid materials.

3.     A method as claimed in claim 1 or 2, wherein a said liposome is selected from phosphatidyl-choline, phosphatidyl serine, phosphatidylinositol, phosphatidylethanolamine, phosphatidic acid, cardiolipin and sphingomyelin.

4.     A method as claimed in any preceding claim, wherein the porin is obtained from the outer membrane of gram negative bacteria of mitochondria.

5.     A method claimed in any preceding claim, wherein the cation is hydrogen ion, alkali metal ion or alkaline earth metal ion.

6.     A method as claimed in Claim 5, wherein the ionophore is selected from valinomycin, gramicidin A, nonactin, monactin dicyclohexyl 18-crown-6, cryptate 211, nigericin, monensin, A 23187, X 537A and alamethecin.

7.     A method as claimed in any preceding claim wherein the ionophore additionally contains a cation different from that which loads the liposomes lacking porins.

8.     A method as claimed in Claim 7 wherein the cation loading the liposomes lacking porins is $K^+$ and that in said medium is $Na^+$.

9.     A method as claimed in any preceding claim wherein the cations loading the liposomes lacking porins are present as aqueous salt solutions having a salt concentration of from 20 to 200 mM.

10.     A mehtod as cliamed in any preceding claim, wherein the fluorescent dye is a a cyanine dye, a merocyanine dye or an oxonal dye.

11.     A method as claimed in any preceding claim, wherein fusion between liposomes containing porins and liposomes lacking porins is initiated by means of a fusogen selected from $Ca^{++}$, polyethylene glycol and Sendai virus or by imposing an electric field.

Fig.I.

Fig.2.

FIG.3.

FIG.4.

3/4

0148551

Fig.5.

FUSION INDEX (%)

TEMPERATURE (°C)

Fig.6.

FUSION INDEX (%)

INCUBATION TIME (min.)

Fig.7.